# EUROPEAN PATENT APPLICATION

(11) **EP 2 213 644 A1**
(43) Date of publication of application: **04.08.2010**
(21) Application number: 09001155.2
(22) Date of filing: 28.01.2009
(51) Int. Cl.: C07C 41/09, C07C 43/188, C07C 43/18, C07C 43/15, C07F 17/02

(54) **Process for the preparation of an allyl alkyl ether by catalyic allylation**

(71) Applicant: Hexion Specialty Chemicals Research Belgium S.A., 1348 Ottignies Louvain-la-Neuve (BE)
(72) Inventor: Postma, Ron, 3196 KK Vondelingenplaat RT (NL)

(57) **Abstract**

The invention provides a process for the preparation of an allyl alkyl ether comprising the allylation of an aliphatic hydroxyl containing compound with an allyl source in the presence of a catalyst, wherein the catalyst is a transition metal complex with a phosphine ligand, wherein the allylation is carried out in the presence of an acid in an amount of at least 0.1 mol% calculated on the aliphatic hydroxyl containing compound.

This invention further provides a process for the preparation of epoxy resins wherein as intermediate use is made of the allyl alkyl ethers prepared by the process of the invention.

## Description

### References cited in the description

*- This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4740330 (Page 1, lines 14, 27 and 29)
- US 7049388 (Page 1, line 23)
- EP 380162 (Page 2, line 27)
- CA 2086285 (Page 2, line 29)
- WO 0187899 (Page 2, line 32)

### Non-patent documents cited in the description

- VAN DER DRIFT, Robert C., et al. Two reactions of allylic alcohols catalysed by ruthenium cyclopentadienyl conplexes with didentate phosphine ligands: isomerisation and ether formation. Journal of Molecular Catalysis A: Chemical. 2000, vo1.159, p.163-177. (Page 1, line 32, page 6, line 32, page 9, line 2)
- TROST, B., et al. Chemoselectivity in the ruthenium-catalyzed redox isomerization of allyl alcohols J. Am. Chem. Soc.. 1993, vol. 115, p.2027-2036. (Page 2, line 8)
- SABURI, Hajime, et al. Catalytic Dehydrative Allylation of Alcohols. Angew. Chem., Int. Ed Engl.. 2005, vol.44, p.1730-1732. (Page 2, line 17)
- Bruce, M. I.; Wong, F. S.; Skelton, B. W.; White, A. H. J. Chem. Soc.-Dalton Trans. 1981, 1398-1405 (Page 9, line 1)
- Alonso, A. G.; Reventos, L. B. J. Organomet. Chem. 1988, 338, 249-254. (Page 9, line 1)

### PROCESS FOR THE PREPARATION OF AN ALLYL ALKYL ETHER BY CATALYTIC ALI-Yl-ATION

### Technical Field

The present invention relates to a process for the preparation of an allyl alkyl ether by catalytic allylation of aliphatic hydroxyl containing compounds. This may be achieved by reaction with an allyl source such as allyl alcohol or a similar reactant in the presence of a suitable catalyst. The catalytic allylation is achieved by the use of metal complexes with certain phosphine ligands in an acidic environment as catalysts.

### Background Art

Allyl ethers of polyols are useful for preparing epoxy compounds low in aliphatic halogen content. From US 4740330 it is known that the aromatic hydroxyl groups of aromatic hydroxyl-containing compounds can be O-allylated to greater than 95% conversion by reacting the alkali metal salt of said aromatic hydroxyl-containing compounds with an allyl halide such as allyl chloride in the presence of a polar aprotic solvent such as dimethylformamide. These compounds are useful in preparing epoxy resins containing a low halogen content. The process of this reference requires the use of an allyl halide and thus generates halide salts as byproducts. From an environmental and atom-efficiency point of view, this route is not attractive.

Likewise US 7049388 discloses a process for manufacturing an alpha -dihydroxy derivative from an aryl allyl ether, and converting such [alpha]-dihydroxy derivative to an epoxy resin. For example, this process is used for manufacturing a bisphenol A epoxy resin.. Thus, in Example 1 the conversion of bisphenol A to bisphenol A diallyl ether is described, which was synthesize as described in US4740330 mentioned above. This process therefore suffers from the same disadvantages as that described in US4740330. The reference does not concern the allylation of the hydrogenated version of bisphenol A or bisphenol-F, which are of commercial interest.

From **VAN DER DRIFT, Robert C., et al.** Two reactions of allylic alcohols catalysed by ruthenium cyclopentadienyl complexes with didentate phosphine ligands: isomerisation and ether formation. Journal of Molecular Catalysis A: Chemical. 2000, vol.159, p.163-177. it is known that ethers may be formed from heterocoupling of allyl alcohol with alcohols. In particular [RuClCp(*o*-MeO-dppe)], wherein Cp stands for η⁵-cyclopentadienyl and *o*-MeO-dppe stands for 1,2-bis(di(*ortho*-methoxyphenyl)phosphino)ethane, has proven to be very active in ether formation. The current inventors set out to improve the efficiency of said catalysts.

In the same article and in a similar article by TROST, B., et al. J. Am. Chem. Soc.. 1993, vo1.115, p.2027-2038 certain ruthenium cyclopentadienyl complexes were reported to be efficient catalysts for the isomerisation of allyl alcohols into aldehydes. These catalysts, based on 1,4-bis(diphenylphosphino)butane (dppb) and on two monodentate phosphine ligands (here triphenylphospine, or PPh₃), were found to be inactive for allylation, resulting in the preparation of propanal from allyl alcohol in an isomerisation reaction. Indeed, in the article of VAN DER DRIFT et al., these catalysts were believed to be completely inactive towards allylation.

In SABURI, Hajime, et al. Catalytic Dehydrative Allylation of Alcohols. Angew. Chem., Int. Ed. Engl.. 2005, vol.44, p.1730-1732. the salt waste-free allylation of various monoalcohols is described, using allyl alcohol as allyl source. Water is thus the only co-product, making this dehydrative allylation both efficient, environmentally friendly and simple to operate. It too employs a cationic ruthenium cyclopentadienyl complex. On the other hand, the direct allylation of the aliphatic alcohols with allyl alcohol (2-propen-1-ol), catalyzed by [CpRuPF₆]-2-pyridinecarboxylic acid derivative combined systems leaves room for improvement, both in terms of yield and conversion speed.

It should also be noted that bidentate phosphines and their synthesis have been disclosed in earlier patent references, such as EP 380162 on catalyst compositions suitable for the preparation of polymers of carbon monoxide and one or more olefinically unsaturated compounds. Likewise, in CA 2086285 a process is found for the preparation of vicinally-disubstituted bis(diorganophosphino)alkanes, -alkenes, or -arenes, which are prepared by reacting a secondary phosphine with a vicinally disubstituted dihaloalkane, -alkene or -arene. Moreover, in WO 0187899 bidentate ligands are described having a bivalent aliphatic bridging group containing from 2 to 6 atoms in the bridge, which is substituted with two or more substituents. The catalyst system comprises: a) a source of group 8-10 metal cations, b) a source of such a bidentate ligand, and c) a source of anions. Also described is the use of such a catalyst system in a process for the carbonylation of optionally substituted ethylenically or acetylenically unsaturated compounds by reaction with carbon monoxide and a co-reactant. None of these references, however, discuss the preparation of allylated aliphatic alcohols.

### Disclosure of Invention

Accordingly, the invention provides a process for the preparation of an allyl alkyl ether comprising the allylation of an aliphatic hydroxyl containing compound with an allyl source in the presence of a suitable catalyst, wherein the catalyst is a complex of a transition metal M with a phosphine ligand, and wherein the phosphine ligand is
- either a monodentate phosphine or a bidentate diphosphine, wherein the allylation is carried out in the presence of an acid in an amount of at least 0.1 mol% calculated on the hydroxyl groups of the aliphatic hydroxyl containing compound.

The monodentate ligand is preferably a phosphine of the formula P(X)₃, wherein each X independently is a an aliphatic substituent having from 1 to 9 carbon atoms, preferably up to 6 carbon atoms, or a cycloaliphatic substituent having from 5 to 9 carbon atoms or aromatic substituent having from 6 to 9 carbon atoms. Two substitutents X may also together form a divalent alkylene group, with from 3 up to 6 carbon atoms, attached to the same phosphorus atom. The cycloaliphatic substituent and/or the aromatic substitutent may be substituted with one or more alkyl groups having up to 6 carbon atoms. These substituents X may also comprise one or more functional groups and/or have one or more heteroatoms as part of the group.

The bidentate ligand is preferably a diphosphine of the formula X₂P-R-PX₂ wherein each X has the same meaning as defined for the monodentate phosphine, wherein the divalent alkylene group may be attached to the same or different phosphorus atom(s), and R is a bridging group, preferably having at least 2 bridging atoms between the phosphorus atoms. R may be a linear bridging group or a substituted (branched) bridging group, carrying one or more substituents on one or more of the bridging atoms. Moreover, R may have carbon atoms in the bridge, but also heteroatoms, such as oxygen and silicon.

More specifically, the invention provides a process for the preparation of an allyl alkyl ether using a ruthenium-cyclopentadienyl complex (Ru-Cp, wherein Cp is a cyclopentadienyl group or a substituted cyclopentadienyl group) as catalyst.

The allyl alkyl ethers prepared by the process of the invention can be used, amongst others, as intermediate in the synthesis of epoxy resins. Hence the use of these ethers has also been claimed.

### Modes for Carrying Out the Invention

Where in this application reference is made to a catalyst, then such definition includes the precatalyst that is activated during the reaction, as well as the activating species in different oxidation states generated during the catalytic reaction. For instance, it is common to activate a Ru-Cp-Cl complex with silver tosylate (4-toluenesulfonate) to remove the strongly coordinating chloride anion present in the precatalyst. Instead of silver tosylate, also silver triflate (trifluoromethanesulfonate), silver tetrafluoroborate, silver hexafluorophosphate and similar non-coordinating silver salts may be used.

The aliphatic hydroxyl containing compound is an alcohol or polyol of the formula R(OH)ₙ, wherein R is a group having at least one hydroxyl group attached thereto. Methanol (CH₃OH) is the simplest species of this genus of (optionally substituted) alkanols. 1,2-ethanediol or 1,2,3-propanetriot are the simplest species carrying more than one hydroxyl group of the genus (optionally substituted) alkanediols and alkanepolyols. The hydroxyl group may be a primary, secondary or tertiary group. Moreover, the aliphatic alcohol may carry one or more inert substituents, such as in polyethylene glycol and sugars (having oxygen atoms in the backbone). Moreover, a significant advantage of the current invention is that the allylation may be carried out at reduced temperatures, opening opportunities for allylation of volatile alcohols such as methanol.

Alcohols that are currently glycidated to prepare epoxy resins and resin modifiers are monools, diols, and triols (e.g., glycerol), that are typically linear and have 3 to 13 carbon atoms. Of particular interest are aliphatic and cycloaliphatic compounds having 2 or more hydroxyl groups, which result in allyl alkyl ethers that are multifunctional and hence suitable as intermediate in the synthesis of epoxy resins. Examples of such compounds include 1,2-ethanediol, 1,2-propanediol, 1,3-propanediol and oligomers and polymers thereof; pentaerythritol; sugars; polybutenediol and similar hydroxyl-terminated oligomers and polymers; optionally substituted cycloalkanols, cycloalkanediols or cycloalkanepolyols; optionally substituted bicycloalkanols; bicycloalkanediols or bicycloalkanepolyols; optionally substituted bis(hydroxycycloalkyl)alkanes and mixtures thereof.

Compounds of more particular interest are selected from one or more of: aliphatic glycols and polyether glycols such as C₂₋₂₄ alkylene glycols and poly(ethylene oxide) or poly(propylene oxide) glycols; an optionally cyclohexanol, cyclohexanediol or cyclohexanepolyol; an optionally substituted bicyclononanol or bicyclodecanol; an optionally substituted bicyclononanediol or bicyclodecanediol; an optionally substituted bicyclononanepolyol or bicyclodecanepolyol; or an optionally substituted bis(hydroxycyclohexyl)-methane, -ethane or propane.

Of known commercial interest are the allylated products made of C₈₋₁₀ alcohols;

C₁₂₋₁₄ alcohols; C₁₂₋₁₃ alcohols; polyethylene glycol; trimethylolethane; dibromo neopentyl glycol; 1-butanol; 1,4-butanediol; neopentyl glycol; cyclohexanedimethanol; bis(hydroxycyclohexyl)propane; castor oil (a triglyceride in which approximately ninety percent of fatty acid chains are ricinoleic acid, having hydroxyl functionality on the ricinoleic acid group); 1,6-hexanediol, and polypropylene glycol. Use of said alcohols is therefore particularly preferred.

The process of the current invention aims at the production of allyl alkyl ethers in an environmentally friendly and atom-efficient manner. As allyl source, allyl halides are preferably excluded, since these would result in the generation of a saline waste. To a lesser extent, also allyl acetates are preferably excluded. Rather, the preferred allyl source is either an allyl alcohol or an allyl ether. 2-Propen-1-ol is again the simplest species of an allyl alcohol. Other examples of suitable allyl alcohols include 3-buten-2-ol, cinnamyl alcohol (3-phenyl-2-propen-1-ol), and other alcohols of the general formula 1-R-2-propen-1-ol or 1-Ar-2-propen-1-ol or 1-RO-2-propen-1-ol or 1-ArO-2-propen-1-ol, or 3-R-2-propen-1-ol or 3-Ar-2-propen-1-ol or 3-RO-2-propen-1-ol or 3-ArO-2-propen-l-ol, wherein R represents an alkyl group, preferably of up to 6 carbon atoms, wherein O represents an oxygen atom, and wherein Ar represents an aryl group, preferably of up to 12 carbon atoms. These alcohols may be used by themselves, or in a mixture. Moreover, their diethers may be used, or ethers of a mixture of these alcohols. Moreover, a mixture of allyl alcohol(s) and allyl ether(s) may be used.

Preferably the allyl source is 2-propen-1-ol ("AA") or the ether thereof ("DAE"), or a combination of AA and DAE, most preferably AA.

The aliphatic hydroxyl containing compound and the allyl source are preferably reacted in amounts sufficient to have at least one of the hydroxyl groups of the aliphatic hydroxyl containing compound react with the allyl source. In case of an aliphatic monool and AA as the allyl source, the molar ratio between these reactants varies from 10:1 to 1:10, suitably from 5:1 to 1:5, preferably from 3:1 to 1:3, more preferably from 2:1 to 1:2. In case of DAE, the relative amount of aromatic hydroxyl containing compound is increased, as DAE may generate ideally two molecules of AA to react with the aliphatic hydroxyl containing compound, In case of a aliphatic polyol (e.g., the saturated versions of bisphenol A or F) the relative amount of aliphatic hydroxyl containing compound is decreased, corresponding to the higher hydroxyl content of said compound.

The catalyst is a transition metal complex with either two monodentate phosphine ligands or a bidentate diphosphine ligand, and optionally comprising a coordinating species and/or a counter ion, wherein M is a transition metal, L is a coordinating species, Y is a counter ion and the specified monodentate or bidentate phosphine ligand.

Suitable transition metals include ruthenium, rhodium, rhenium, palladium, iridium, tungsten, molybdenum, chromium, platinum, nickel, tin, copper, osmium, iron, and preferably from ruthenium, osmium, and iron and more preferably is ruthenium. Various coordinating species L may be used, subject to the selection of the transition metal and the transition state of the metal. It is reiterated that the transition state of the metal as active catalyst may and frequently is different from that of the precatalyst. Coordinating species may include carbonyl groups, indenyl, cyclopentadienyl and optionally substituted cyclopentadienyl groups. Indeed, in case of a ruthenium complex, the coordinating species is preferably cyclopentadienyl or a substituted cyclopentadienyl group (e.g., Cp* = pentamethylcyclapentadienyl).

From VAN DER DRIFT et al., mentioned supra, bidentate ligands and Ru-Cp complexes based thereon are known. Surprisingly, it has been discovered that in the presence of strong acid, for instance p-toluenesulfonic acid, the complexes become very active in catalyzing allylation reactions.

In case of a bidentate diphosphine, the bridge may comprise two or more bridging atoms. Preferably, the bridge comprises 2 to 4 carbon atoms, i.e., a 1,4-butylene group, 1,3-propylene group, or-preferably- a 1,2-ethylene group. Also suitable are bidentate diphosphines wherein the bridging group is branched or substituted, as in e.g., 1,2-propylene, etc. The substituent on the bridging group should preferably have no more than 8 atoms.

Preferably the substituents X are identical.

Illustrative examples of suitable ligands therefore include.
- 1,2-bis(diphenylphosphino)-ethane (dppe)
- 1,4-bis(diphenylphosphino)-butane (dppb) and
- triphenylphosphine.

The catalyst is present during the reaction in a catalytically effective amount, preferably in an amount of from about 0.005 to about 200 mmoles of metal per mole of hydroxyl group(s) in the aliphatic hydroxyl containing compound. More preferably, the catalyst is used in an amount of from about 0.05 to 20 mmoles per mole of hydroxyl group(s) in the aliphatic hydroxyl containing compound. Indeed, for improved activity, most preferably small amounts of catalyst are used, such as about 0.1 mmole per mole of hydroxyl group(s) in the aliphatic hydroxyl containing compound.

As mentioned before, the catalyst may need to be activated to perform the O-allylation. Ru-Cp complexes typically have a strongly coordinating chloride anion when obtained as precatalyst, which may be removed with silver tosylate to create a vacant site for substrate coordination.

The catalyst may be immobilized, resulting in a heterogeneous catalyst. For instance, the catalyst (metal complex) can be immobilized on an ion-exchange resin with sulfonic acid groups. Examples of suitable resins include for example DOWEX™ WX4, or Amberlys™ 15, or Nafion™ NR 50.

The O-allylation products may be prepared in a continuous reaction or in a batch reaction.

In the process of the current invention, the allylation reaction is performed in the presence of an acid. Thus, the inventors surprisingly found a high activity in the process of the invention when performed in the presence of an acid. Thus, the presence of 0.1 mol% to 25 mol%, preferably 0.15 to 20 mol%, more preferably 0.2 to 10 mol% and most preferably 1 to 5 mol% of acid on the number of hydroxyl groups in the aliphatic hydroxyl containing compound increases the allylation reaction rate. The acid may be selected on the basis of its logarithmic acid dissociation constant, pKa. The acid is selected preferably from organic acids with a pKa < 5, preferably with a pKa < 3. Suitable acids include acetic acid and triflic acid and such, an in particular those acids whose silver salt have been used to activate the catalyst. The most preferred acid is p-toluenesulfonic acid.

The reaction is preferably carried out in an organic solvent. Although solvents like diethylene glycol dimethyl ether (diglyme) may be used, the solvent is more preferably an apolar solvent. The selection typically is done on the basis of the availability, cost and acceptability for environmental and health issues. The solvent has an effect on the total conversion rate of the reaction. The organic solvents may be selected from aromatic or aliphatic hydrocarbons, with the preferred solvent being toluene and the most preferred solvent being n-heptane.

The reactions are preferably conducted at a temperature between 40 and 150°C, more preferably between 80 and 130°C, still more preferably between 90 and 110°C.

The following examples will more fully illustrate selected embodiments of this invention. All parts, percentages and proportions referred to herein and in the appended claims are by mol unless otherwise indicated.

### Examples

**Table 1, ligand abbreviations**

| Abbreviation | Compound |
|---|---|
| dppe | 1,2-bis(diphenylphosphino)ethane |
| dppb | 1,4-bis(diphenylphosphino)butane |
| PPh₃ - | triphenylphosphine |

### Ligand synthesis

The ligands were commercially available from Acros.

The ruthenium complexes (RuCpCl(PPh₃)₂]¹; [RuCpCl(dppe)]² and [RuCpCl(dppb)]³ were synthesized as reported.

### Reaction of cyclohexanol with allyl alcohol under catalysis of different complexes

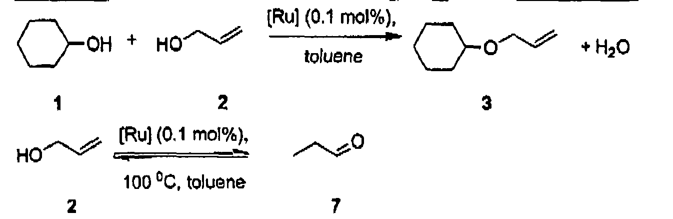

Allylation reactions were carried out in the manner shown above, at a molar ratio of cyclohexanol/allyl alcohol/catalyst/AgOTs = 1000/1000/1/2, using toluene as solvent, at 100 °C for 3 hours, unless otherwise specified. The products were analyzed by GC, identified by GG-MS and NMR spectroscopy and the results are reported below.

### Comparative Example 1

[RuCpCl(dppe)]. 2.5 mmol of cyclohexanol, 2.5 µmol of [RuCpCl(dppe)] and 5 µmol of AgOTs were charged into the reaction vessel and flushed with argon. Degassed and dried toluene was added (3 ml) and the mixture was stirred for five minutes. Allyl alcohol was added (2.5 mmol) and the reaction was stirred for 3 hours at 80 °C. Yield of 3: 8%.

### Comparative Example 2

[RuCpCl(dppe)]. As example 1, but at 100°C. Yield of 3: 33%.

### Comparative Example 3

[RuCpCl(dppb)], As example 2, but with [RuCpCl(dppb)) instead of [RuCpCl(dppe)]. Yield of 3: 0%. Meanwhile the conversion of 2 into 7 is 100%.

### Comparative Example 4

(RuCpCl(dppe)]. As example 2, but with 5.0 instead of 2.5 mmol of allyl alcohol, and after 1 hour. Yield of 3: 28%.

### Comparative Example 5

[RuCpCl(PPh₃)₂]. As example 4, but with [RuCpCl(PPh₃)₂] instead of [RuCpCl(dppe)]. Yield of 3: 0%. Meanwhile the conversion of 2 into 7 is 100%.

¹ Bmcc, M.I.; Wong, F. S.; Skelton, B. W.; White, A. H. J. Chem. Soc.-Dalton Trans. 1981, 1398-1405.

² Alonso, A. G.; Reventos, L. B. J. Organomet. Chem. 1988, 338, 249-254.

³ VAN DER DRIFT, Robert C., et al. Two reactions of allylic alcohols catalysed by ruthenium cyclopentadienyl conplexes with didentate phosphine ligands: isomerisation and ether formation. Journal of Molecular Catalysis A: Chemical. 2000, vol.159, p.163-177.

### Example 6

[RuCpCl(dppe)]. As example 1, (80°) but with 5.0 µmol of *p*-toluenesulfonic acid. Yield of 3:40%.

### Examples 7

(RuCpCl(dppe)]. As example 2, (100°) but with 5.0 µmol of *p*-toluenesulfonic acid. Yield of 3: 72%.

### Example 8

[RuCpCl(dppb)). As example 3, but with 5.0 µmol of *p*-toluenesulfonic acid. Yield of 3: 25%.

### Example 9

[RuCpCl(dppe)]. As example 4, but with 5.0 µmol of *p*-toluenesulfonic acid. Yield of 3: 48%.

### Example 10

[RuCpCI(PPh₃)₂]. As example 5, but at with 50.0 µmol of *p*-toluenesulfonic acid. Yield of 3: 17%. (Lesser amounts of acid resulted in increased amount of 7).

### Example 11

[RuCpCl(dppe)]. As example 9, but n-heptane instead of toluene. Yield of 3: 51 %. Conclusions/observations

Comparative examples 1 to 5 are without acid. The yield of 3 is low, if any at all. In case of [RuCpCl(dppb)] there is no reaction at al, for the other catalyst (some) conversion of the allyl alcohol into its diether is observed.

Examples 6 to 9 (bidentate ligands) are identical to comparative examples 1 to 4, but with the addition of 5.0 µmol of *p*-toluenesulfonic acid. Likewise, Example 10 (monodentate ligand) is identical to comparative example 5, but with the addition of 50.0 µmol of *p*-toluenesulfonic acid. In each instance the yield of 3 improved (significantly).

Example 7, when compared to example 6, illustrates that a temperature of 100°C is preferred.

Example 10 illustrates that the positive effect of the acid is found not only for bidentate ligands, but also for monodentate ligands. On the other hand, the results also illustrate that dppe is the more efficient ligand than dppb, and that dppb is the more efficient ligand than PPh₃.

Finally, Example 11 illustrates that the positive effect of n-heptane as solvent. Thus, the yield of 3 was improved from 48% (toluene) to 51 % (n-heptane).

### Industrial Application

Intermediate for pharmaceuticals, e.g., wherein the allylation reaction is applied to provide a protecting group on a hydroxyl functionality in multistep synthesis of certain fine and/or pharmaceutical compounds.

Intermediate for epoxy resins

## Claims

1. A process for the preparation of an allyl alkyl ether comprising the allylation of an.aliphatic hydroxyl containing compound with an allyl source in the presence of a suitable catalyst, wherein the catalyst is a complex of transition metal M with a phosphine ligand, wherein the allylation is carried out in the presence of an acid in an amount of at least 0.1 mol% calculated on the aliphatic hydroxyl containing compound.

2. The process of claim 1 wherein the phosphine ligand is
- either a monodentate phosphine, preferably a phosphine of the formula P(X)₃, wherein each X independently is a an aliphatic substituent having from 1 to 9 carbon atoms, or a cycloaliphatic substituent having from 5 to 9 carbon atoms or an aromatic substituent having from 6 to 9 carbon atoms, or wherein two substituents X together form a divalent alkylene group, with from 3 up to 6 carbon atoms, ,
- or a bidentate diphosphine, preferably a diphosphine of the formula X₂P-R-PX₂ wherein each X has the same meaning as defined for the monodentate phosphine, wherein the divalent alkylene group is attached to the same or different phosphorus atom(s), and R is a bridging group having at least 2 bridging atoms between the phosphorus atoms.

3. The process of claim 1 or 2, wherein M is selected from the transition metals ruthenium, rhodium, rhenium, palladium, iridium, tungsten, molybdenum, chromium, platinum, nickel, tin, copper, osmium, iron, and preferably from ruthenium, osmium, and iron and more preferably is ruthenium.

4. The process of claim 3, wherein a ruthenium-cyclopentadienyl complex (Ru-Cp, wherein Cp is a cyclopentadienyl group or a substituted cyclopentadienyl group) is used as catalyst.

5. The process of any one of claims 1 to 4, wherein the phosphine ligand is a bidentate diphosphine as defined in claim 2, wherein the bridging group R is substituted or branched.

6. The process of any one of claims 1 to 5, wherein the substituents X attached to the phosphorus atom(s) are independently selected from: phenyl, ortho-methoxyphenyl, ortho-ethoxyphenyl, and cyclohexyl.

7. The process of any one of claims 1 to 6, wherein the ligand is one of:
1,2-bis(diphenylphosphino)ethane (dppe):
1,4-bis(diphenylphosphino)-butane (dppb); or
triphenylphosphine.

8. The process of any one of claims 1 to 7, wherein the catalyst is present during the reaction in a catalytically effective amount, preferably in an amount of from about 0.005 to about 200 mmoles of metal per mole of hydroxyl group(s) in the aliphatic hydroxyl containing compound.

9. The process of any one of claims 1 to 8, wherein the aliphatic hydroxyl containing compound is a compound comprising an aliphatic or cycloaliphatic group having at least one hydroxyl group attached thereto, preferably a primary or a secondary hydroxyl group, or a mixture of compounds.

10. The process of claim 9, wherein the aliphatic hydroxyl containing compound is an aliphatic glycol or polyether glycol; an optionally substituted alkanol, alkanediol or polyol; an optionally substituted cycloalkanol, cycloalkanediol or cycloalkanepolyol; an optionally substituted bicycloalkanol; bicycloalkanediol or bicycloalkanepolyol; an optionally substituted bis(hydroxycycloalkyl)alkane.

11. The process of any one of claims 1 to 10, wherein the allyl source is an allyl alcohol or mixture of allyl alcohols of the general formulae
1-R-2-propen-1-ol or
1-Ar-2-prapen-1-ol or
1-RO-2-propen-1-ol or
1-ArO-2-propen-1-ol, or
3-R-2-propen-1-ol or
3-Ar-2-propen-1-ol or
3-RO-2-propen-1-ol or
3-ArO-2-propen-1-ol,
wherein R represents an alkyl group, preferably of up to 6 carbon atoms,
wherein O represents an oxygen atom, and wherein Ar represents an aryl group, preferably of up to 12 carbon atoms and/or an ether of the allyl alcohol or allyl alcohols, preferably 2-propen-1-ol ("AA") or the ether thereof ("DAE") or the mixture of AA and DAE, preferably AA.

12. The process of any one of claims 1 to 11, wherein the allyl alcohol and the aliphatic hydroxyl containing compound are used in a molar ratio of allyl alcohol versus the aliphatic hydroxyl-containing compound of from 1:10 to 10:1, preferably of from 1:5 to 5:1, more preferably of from 1:2 to 2:1, most preferably about 1:1.

13. The process of any one of claims 1 to 12, wherein an acid with pKa < 5, preferably a pKa <3, preferably selected from acetic acid, triflic acid and p-toluenesulfonic acid, more preferably p-toluenesulfonic acid is present in an amount of 0.1 mol% to 25 mol%, preferably 0.15 to 20 mol%, more preferably 0.2 to 10 mol% and most preferably 1 to 5 mol% calculated on the amount of aliphatic hydroxyl containing compound.

14. The process of any one of claims 1 to 13, conducted at a temperature between 40 and 150°C, preferably between 80 and 130°C more preferably between 90 and 110°C.

15. Process for the preparation of epoxy resins wherein as intermediate use is made of the allyl alkyl ethers prepared by the process of any one of claims 1 to 14.
